(19) Europäisches Patentamt
European Patent Office
Office européen des brevets

(11) EP 4 063 413 A1

(12) EUROPEAN PATENT APPLICATION
published in accordance with Art. 153(4) EPC

(43) Date of publication:
**28.09.2022 Bulletin 2022/39**

(21) Application number: **21872871.5**

(22) Date of filing: **17.09.2021**

(51) International Patent Classification (IPC):
***C08F 251/00*** $^{(2006.01)}$ ***C08B 31/00*** $^{(2006.01)}$
***C08B 37/08*** $^{(2006.01)}$ ***A61L 15/60*** $^{(2006.01)}$

(52) Cooperative Patent Classification (CPC):
**A61L 15/60; C08B 31/00; C08B 37/003; C08F 251/00**

(86) International application number:
**PCT/KR2021/012862**

(87) International publication number:
**WO 2022/065843 (31.03.2022 Gazette 2022/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.09.2020 KR 20200125238**

(71) Applicant: **Lg Chem, Ltd.**
**Seoul 07336 (KR)**

(72) Inventors:
- **CHOI, Hyungsam**
  **Daejeon 34122 (KR)**
- **YUN, Haesung**
  **Daejeon 34122 (KR)**
- **HAM, Kyungrok**
  **Daejeon 34122 (KR)**
- **CHO, Beomshin**
  **Daejeon 34122 (KR)**
- **LEE, Donghwan**
  **Daejeon 34122 (KR)**
- **LEE, Ji Myeong**
  **Daejeon 34122 (KR)**
- **JUNG, Seonjung**
  **Daejeon 34122 (KR)**
- **KANG, Soonhee**
  **Daejeon 34122 (KR)**

(74) Representative: **Goddar, Heinz J.**
**Boehmert & Boehmert**
**Anwaltspartnerschaft mbB**
**Pettenkoferstrasse 22**
**80336 München (DE)**

# (54) BIODEGRADABLE SUPER ABSORBENT POLYMER AND PREPARATION METHOD THEREFOR

(57) The present disclosure provides a biodegradable super absorbent polymer that exhibits excellent biodegradability without deterioration in physical properties of the super absorbent polymer such as centrifuge retention capacity and absorbency under pressure, and a method for producing the same.

EP 4 063 413 A1

[FIG. 2]

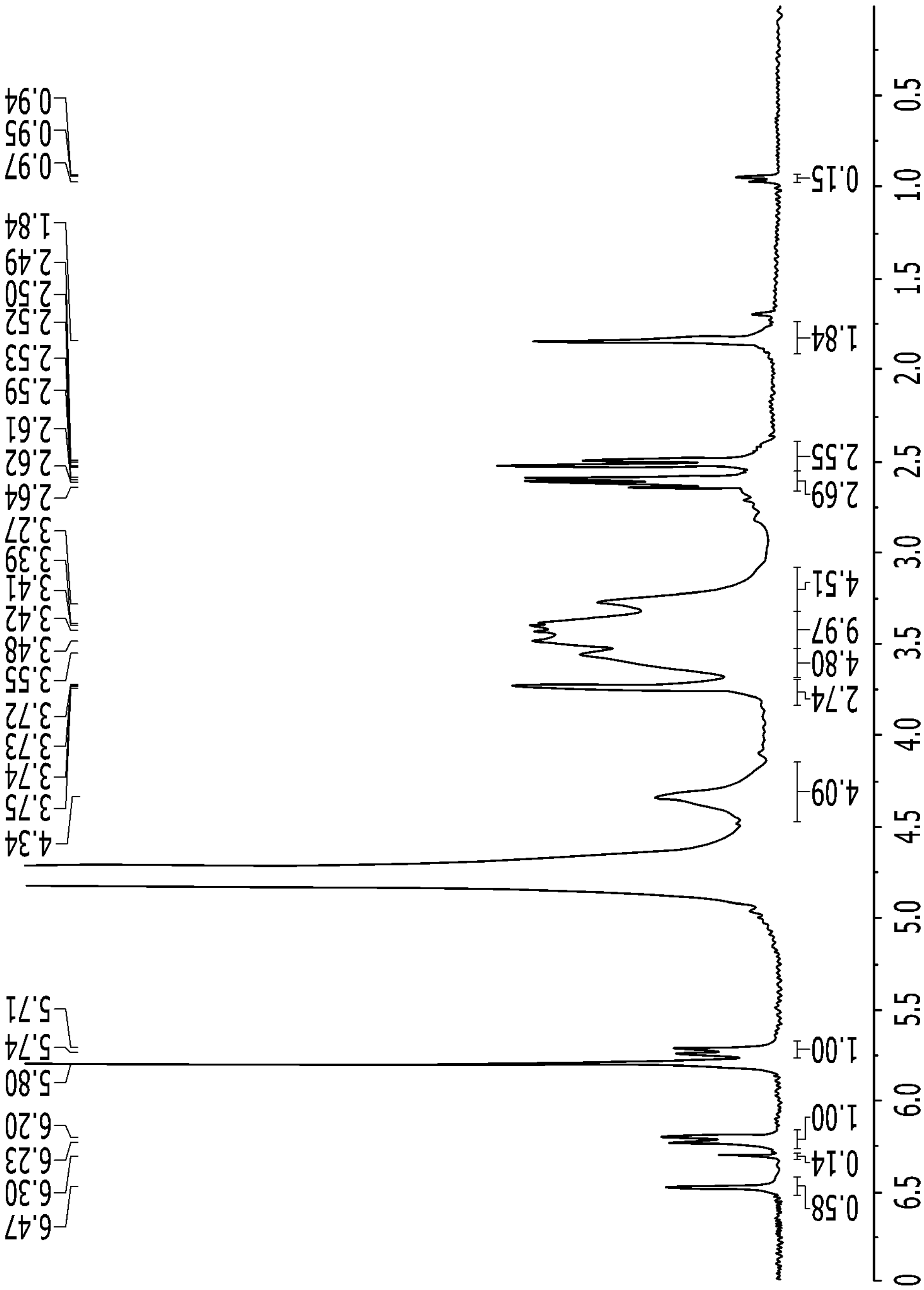
0.94
0.95
0.97
1.84
2.49
2.50
2.52
2.53
2.59
2.61
2.62
2.64
3.27
3.39
3.41
3.42
3.48
3.55
3.72
3.73
3.74
3.75
4.34
5.71
5.74
5.80
6.20
6.23
6.30
6.47
0.15
1.84
2.55
2.69
4.51
9.97
4.80
2.74
4.09
1.00
1.00
0.14
0.58
0.5
1.0
1.5
2.0
2.5
3.0
3.5
4.0
4.5
5.0
5.5
6.0
6.5
0

## Description

### [TECHNICAL FIELD]

CROSS-REFERENCE TO RELATED APPLICATION(S)

This application claims the benefit of Korean Patent Application No. 10-2020-0125238 filed on September 25, 2020 with the Korean Intellectual Property Office, the disclosure of which is incorporated herein by reference in its entirety.

The present disclosure relates to a biodegradable super absorbent polymer that exhibits excellent biodegradability without deterioration in physical properties of the super absorbent polymer such as centrifuge retention capacity and absorbency under pressure, and a method for producing the same.

### [BACKGROUND ART]

A super absorbent polymer (SAP) is a type of synthetic polymeric materials capable of absorbing moisture from about 500 to 1000 times its own weight. Various manufacturers have denominated it as different names, such as SAM (Super Absorbency Material), AGM (Absorbent Gel Material), etc. Since such super absorbent polymers started to be practically applied in sanitary products, now they have been widely used not only for hygiene products such as disposable diapers for children, etc., but also for water retaining soil products for gardening, water stop materials for the civil engineering and construction, sheets for raising seedling, fresh-keeping agents for food distribution fields, materials for poultice or the like.

Such a super absorbent polymer is typically produced after a hydrogel polymer is obtained by subjecting an acrylic acid-based monomer to bulk polymerization or suspension polymerization together with a crosslinking agent in the presence of a polymerization initiator. Therefore, most conventional super absorbent polymers have little biodegradability, which causes environmental problems when performing waste disposal. Specifically, when several products to which super absorbent polymer is applied are buried and disposed, such super absorbent polymer is not decomposed by bacteria or microorganisms in the ground, which may cause environmental pollution.

In view of the above, attempts have been made on super absorbent polymers that exhibit excellent biodegradability using biomass-derived materials, but it has not been easy to produce a biodegradable super absorbent polymer that can be economically produced while exhibiting various physical properties comparable to those of conventional super absorbent polymers.

Accordingly, there is a continuous demand for the development of a super absorbent polymer-related technology capable of exhibiting biodegradability without deteriorating the basic physical properties of the super absorbent polymer.

### [DETAILED DESCRIPTION OF THE INVENTION]

### [Technical Problem]

It is an object of the present disclosure to provide a biodegradable super absorbent polymer that exhibits excellent biodegradability without deterioration in physical properties of the super absorbent polymer such as centrifuge retention capacity and absorbency under pressure, and a method for producing the same.

### [Technical Solution]

According to one embodiment of the present disclosure, there is provided a biodegradable super absorbent polymer comprising:

a crosslinked polymer of a monomer including a modified polysaccharide having a maleic acid group (-OCOCH=CHCOOH) and a sulfosuccinic acid group (-OCOCH($SO_3H$)$CH_2$COOH), and an internal crosslinking agent,
wherein at least a part of the maleic acid group and the sulfosuccinic acid group is neutralized,
the degree of substitution of maleic acid group in the modified polysaccharide is 0.15 to 0.65, and
the degree of substitution of sulfosuccinic acid group in the modified polysaccharide is 0.05 to 0.60.

According to another embodiment of the present disclosure, there is provided a method for producing a biodegradable super absorbent polymer, the method comprising the steps of:

preparing a monomer composition including a modified polysaccharide having a maleic acid group (-OCOCH=CHCOOH) and a sulfosuccinic acid group (-OCOCH($SO_3H$)$CH_2$COOH) in the presence of an internal crosslinking

agent and a polymerization initiator;
subjecting the monomer composition to crosslinking polymerization to produce a hydrogel polymer;
drying, pulverizing and classifying the hydrogel polymer,
wherein at least a part of the maleic acid group and the sulfosuccinic acid group is neutralized,
the degree of substitution of maleic acid group in the modified polysaccharide is 0.15 to 0.65, and
the degree of substitution of sulfosuccinic acid group in the modified polysaccharide is 0.05 to 0.60.

According to yet another embodiment of the present disclosure, there is provided a hygiene product comprising the above-mentioned biodegradable super absorbent polymer.

**[ADVANTAGEOUS EFFECTS]**

The biodegradable super absorbent polymer according to the present disclosure exhibits excellent biodegradability by using a chemically modified polysaccharide. Also, the biodegradable super absorbent polymer of the present disclosure uses a modified polysaccharide having a weight average molecular weight of a certain level or higher and thus, can be subjected to polymerization and crosslinking without unreacted modified polysaccharide, so that general physical properties of the super absorbent polymer such as centrifuge retention capacity and absorbency under pressure may not be reduced. Therefore, the biodegradable super absorbent polymer may not cause a problem of environmental pollution at the time of disposal even if it is applied to various hygiene products.

**[BRIEF DESCRIPTION OF THE DRAWINGS]**

Fig. 1 shows a $^1$H NMR spectrum of maleated chitosan produced in step 1 of Production Example 1; and
Fig. 2 shows a $^1$H NMR spectrum of modified chitosan (M/S) finally produced in Production Example 1.

**[DETAILED DESCRIPTION OF THE EMBODIMENTS]**

The terms used herein are used only to explain illustrative embodiments, and are not intended to limit the scope of the invention. The singular expression is intended to include the plural meaning, unless the context clearly indicates otherwise. As used herein, the terms "comprise", "include", or "have" defines the presence of stated features, integers, steps, components or their combination, but do not exclude the presence or addition of one or more other features, integers, steps, components or their combination.
Also, as used herein, in case a layer or an element is mentioned to be formed "on" or "above" layers or elements, it means that the layer or element is directly formed on the layers or elements, or it means that other layers or elements can be additionally formed between the layers, on a subject, or on a substrate.
Although various modifications can be made to the present disclosure and the present disclosure may have various forms, specific embodiments thereof will be illustrated and described in detail. It should be understood, however, that there is no intent to limit the invention to the particular forms disclosed, and the invention is to cover all modifications, equivalents, and alternatives without departing from the spirit and scope of the invention.
Further, the technical terms used herein is only for reference to specific embodiments only, and is not intended to limit the present disclosure. And, the singular terms used herein also include plural terms unless phrases clearly express opposite meanings.
Meanwhile, the term "(meth)acrylate" as used herein includes not only acrylate but also methacrylate.
As used herein, the term "polymer" or "macromolecular" means the polymerized state of water-soluble ethylenically unsaturated monomers, and may encompass those of all moisture content ranges or particle diameter ranges. Among the polymers, those having water content (moisture content) of about 40 wt. % or more after polymerized and before dried may be referred to as hydrogel polymer. The particles in which the hydrogel polymer is pulverized and dried may be referred to as a crosslinked polymer.
Further, the term "super absorbent polymer particles" refers to particulate matters including a crosslinked polymer in which a modified starch containing an acidic group, having an acidic group and a vinyl group and having at least partially neutralized acidic groups is polymerized, and crosslinked by an internal crosslinking agent.
Further, the term "super absorbent polymer" means a cross-linked polymer in which a modified starch having an acidic group and a vinyl group according to the context and having at least partially neutralized acidic groups is polymerized together with an internal crosslinking agent, or a base polymer in the form of powder composed of super absorbent polymer particles in which the crosslinked polymer is pulverized, or it is used to comprehensively include those made to be appropriate for productization through additional processes of the crosslinked polymer or base polymer,

such as surface crosslinking, particle reassembly, drying, pulverization, sieving, and the like.

The super absorbent polymer that is commonly used in the art is produced by polymerizing an acrylic acid-based monomer together with a crosslinking agent in the presence of a polymerization initiator, but the super absorbent polymer produced in this way does not have biodegradability, which causes an environmental problem.

In view of the above, development has been made for a super absorbent polymer that can exhibit biodegradability. As a biodegradable material capable of producing such a biodegradable polymer, materials such as polysaccharide, polyaspartic acid, polyglutamic acid, etc. have been discussed, but these have reduced the absorption capacity, which is an important physical property of the super absorbent polymer, and thus there have been difficulties in replacing the super absorbent polymer produced with an acrylic acid-based monomer.

Therefore, the present inventors have found that when a super absorbent polymer is produced by using a modified polysaccharide having a maleic acid group (-OCOCH=CHCOOH) and a sulfosuccinic acid group (-OCOCH($SO_3H$)$CH_2$COOH) as a monomer, not only the biodegradability is excellent, but also various physical properties of super absorbent polymers, such as centrifuge retention capacity and absorbency under pressure are excellent compared to those of known biodegradable materials, thereby completing the present disclosure.

Hereinafter, a biodegradable super absorbent polymer and a method for producing the same according to specific embodiments of the present disclosure will be described in more detail.

**Biodegradable super absorbent polymer**

Specifically, the biodegradable super absorbent polymer according to one embodiment of the present disclosure includes a crosslinked polymer of a monomer including a modified polysaccharide having a maleic acid group (-OCOCH=CHCOOH) and a sulfosuccinic acid group (-OCOCH($SO_3H$)$CH_2$COOH), and an internal crosslinking agent,

Here, the crosslinked polymer is a polymer in which a monomer including a modified polysaccharide having at least partially neutralized maleic acid group (-OCOCH=CHCOOH) and sulfosuccinic acid group (-OCOCH($SO_3H$)$CH_2$COOH) is subjected to a crosslinking polymerization in the presence of an internal crosslinking agent. The crosslinked polymer has a three-dimensional network structure in which the main chains formed by polymerizing polysaccharides are crosslinked by the internal crosslinking agent. When the crosslinked polymer has a three-dimensional network structure in the form in which main chains formed by polymerizing the polysaccharides are crosslinked by the internal crosslinking agent in this way, the centrifuge retention capacity and absorbency under pressure, which are various physical properties of the super absorbent polymers, can be significantly improved compared to the case of a two-dimensional linear structure that is not additionally crosslinked by an internal crosslinking agent.

Meanwhile, the polysaccharide refers to a polymeric carbohydrate molecule composed of glucose repeating units. In this case, the polysaccharide is also referred to including a polymer molecule composed of a glucosamine unit in which an amino group is introduced into a hydroxy group bonded to the 2nd carbon atom within the glucose repeating unit, and/or an N-acetylglucosamine unit in which an N-acetylamino group is introduced into a hydroxy group bonded to the 2nd carbon atom within the glucose repeating unit. Therefore, as the polysaccharide, any compound usually known as a polysaccharide can be used without limitation. Examples thereof include, but are not limited to, starch composed of glucose repeating units, chitosan composed of glucosamine repeating units and N-acetylglucosamine repeating units, and the like.

Further, the modified polysaccharide used in the biodegradable super absorbent polymer is a concept distinguished from unmodified polysaccharides that are usually obtained naturally or synthetically, and means that the hydroxyl group (-OH) in the glucose repeating unit forming the polysaccharide is substituted with another functional group by chemical treatment and/or heat treatment. Such a modified polysaccharide can exhibit different physical properties from the unmodified polysaccharide due to a functional group introduced instead of a hydroxyl group (-OH).

For example, the modified polysaccharide is modified starch or modified chitosan.

Meanwhile, maleic acid group (-OCOCH=CHCOOH) and sulfosuccinic acid group (-OCOCH($SO_3H$)$CH_2$COOH) are substituted in the modified polysaccharide, and at least a part of the maleic acid group (-OCOCH=CHCOOH) and sulfosuccinic acid group (-OCOCH($SO_3H$)$CH_2$COOH) is neutralized.

Further, the maleic acid group (-OCOCH=CHCOOH) and sulfosuccinic acid group (-OCOCH($SO_3H$)$CH_2$COOH) may be introduced into the hydroxyl group of the 6th carbon of the glucose/glucosamine/N-acetylglucosamine repeating unit within the polysaccharide. This is because the hydroxyl group bonded to the 6th carbon has better reactivity than the hydroxyl group bonded to the 2nd and 3rd carbons. Here, the hydroxyl group of the 6th carbon means a hydroxyl group that is bonded to carbon represented by the 6th when representing the glucose repeating unit of a general polysaccharide by the following Chemical Formula A.

[Chemical Formula A]

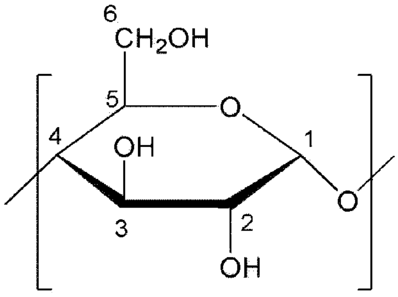

Further, the maleic acid group (-OCOCH=CHCOOH) in the polysaccharide may be introduced by maleic acid or maleic acid anhydride. For example, due to maleic anhydride, the hydroxyl group (-OH) present in an unmodified polysaccharide molecule can be substituted with a maleic acid group (-OCOCH=CHCOOH).

At this time, the degree of substitution (DS) of the maleic acid group (-OCOCH=CHCOOH) of the modified polysaccharide is represented by "$DS_M$" in the following, which ranges from 0.15 to 0.65. When the degree of substitution of the maleic acid group is less than 0.15, a large amount of modified polysaccharides that do not participate in crosslinking polymerization may remain, and when the degree of substitution of the maleic acid group is greater than 0.65, maleic anhydride, which is used in a large amount for introducing a maleic acid group, may not be completely removed, which may affect the physical properties of the final super absorbent polymer. Preferably, the degree of substitution of the maleic acid group is 0.16 or more, 0.17 or more, 0.18 or more, 0.19 or more, 0.20 or more, 0.21 or more, 0.22 or more, 0.23 or more, 0.24 or more, 0.25 or more, 0.26 or more, 0.27 or more, 0.28 or more, 0.29 or more, 0.30 or more, 0.31 or more, 0.32 or more, 0.33 or more, 0.34 or more, 0.35 or more, or 0.36 or greater; and 0.64 or less, 0.63 or less, 0.62 or less, 0.61 or less, 0.60 or less, 0.59 or less, 0.58 or less, 0.57 or less, 0.56 or less, 0.55 or less, 0.54 or less, 0.53 or less, 0.52 or less, or 0.51 or less.

Here, the degree of substitution of the maleic acid group means the average number of hydroxyl groups (-OH) substituted with maleic acid groups per glucose repeating unit. That is, since three hydroxyl groups are present per glucose repeating unit, the theoretical maximum degree of substitution is 3. "The degree of substitution is 0.1" means that one hydroxyl group is substituted per 10 glucose repeating units. The degree of substitution of the maleic acid group can be calculated through the $^1H$ NMR analysis of the finally produced modified polysaccharide. For more detailed contents, refer to Production Examples described later.

Further, the sulfonic acid group in the polysaccharide can be introduced by at least one sulfite-based compound selected from the group consisting of sodium bisulfite, potassium bisulfite, ammonium sulfite and sodium sulfite. For example, the sulfonic acid group may be introduced using sodium bisulfite.

Preferably, the sulfonic acid group may be introduced in the form of a sulfosuccinic acid group ($-OCOCH(SO_3H)CH_2COOH$). In other words, the sulfonic acid group may be introduced in the form of a sulfosuccinic acid group by a reaction between the double bond of the maleic acid group introduced first and the sulfide-based compound.

The degree of substitution of the sulfosuccinic acid group ($-OCOCH(SO_3H)CH_2COOH$) of the modified polysaccharide is represented by "DSs" in the following, which ranges from 0.05 to 0.60. When the degree of substitution of the sulfosuccinic acid group is less than 0.05, there may be a limit to improvement of centrifuge retention capacity and effective absorption capacity, and when the degree of substitution of the sulfosuccinic acid group is greater than 0.60, the number of vinyl groups participating in polymerization is reduced, which may make it difficult to secure a three-dimensional crosslinked polymer structure. Preferably, the degree of substitution of the sulfosuccinic acid group is 0.06 or more, 0.07 or more, 0.08 or more, 0.09 or more, 0.10 or more, 0.11 or more, 0.12 or more, 0.13 or more, 0.14 or more, 0.15 or more, or 0.16 or more; and 0.59 or less, 0.58 or less, 0.57 or less, 0.56 or less, 0.55 or less, 0.54 or less, 0.53 or less, 0.52 or less, 0.51 or less, 0.50 or less, 0.49 or less, 0.48 or less, 0.47 or less, 0.46 or less, 0.45 or less, 0.44 or less, 0.43 or less, 0.42 or less, 0.41 or less, 0.40 or less, 0.39 or less, 0.38 or less, 0.37 or less, 0.36 or less, 0.35 or less, 0.34 or less, 0.33 or less, 0.32 or less, 0.31 or less, 0.30 or less, 0.29 or less, or 0.28 or less.

Preferably, the sum of the degree of substitution of the maleic acid group and the degree of substitution of the sulfosuccinic acid group ($DS_M+DS_S$) is 1 or less, and more preferably 0.2 or more, 0.3 or more, 0.4 or more, or 0.5 or more; and 0.90 or less, 0.85 or less, 0.80 or less, 0.75 or less, or 0.70 or less.

Preferably, the ratio ($DS_S:DS_M$) of the degree of substitution of the maleic acid group and the degree of substitution of the sulfosuccinic acid group within the modified polysaccharide is 1:0.1 to 1:3.0, more preferably 1:0.2 or more, or 1:0.3 or more; 1:2.5 or less, 1:2.4 or less, 1:2.3 or less, 1:2.2 or less, 1:2.1 or less, 1:2.0 or less, 1:1.9 or less, 1:1.8 or less, 1:1.7 or less, 1:1.6 or less, 1:1.5 or less, 1:1.4 or less, 1:1.3 or less, 1:1.2 or less, 1:1.1 or less, 1:1.0 or

less, 1:0.9 or less, or 1:0.8 or less.

Meanwhile, a part of the maleic acid groups and sulfosuccinic acid groups of the modified polysaccharide is neutralized, and the degree of neutralization thereof can be adjusted according to the type of the modified polysaccharide and the physical properties of the final super absorbent polymer to be realized. For example, in the case of modified starch, it has high molecular weight and thus allows water insolubility or neutralization degree to increase, so that the carboxyl group (COOH) can be neutralized to the carboxylate (COO-) form, thereby increasing the solubility in water. Specifically, the degree of neutralization of the modified polysaccharide may be 40 to 95 mol%, or 40 to 80 mol%, or 45 to 75 mol%.

In one embodiment, the modified polysaccharide may include at least one of the repeating units represented by the following Chemical Formulas 1-1 to 1-3; and at least one of repeating units represented by the following Chemical Formulas 2-1 to 2-3.

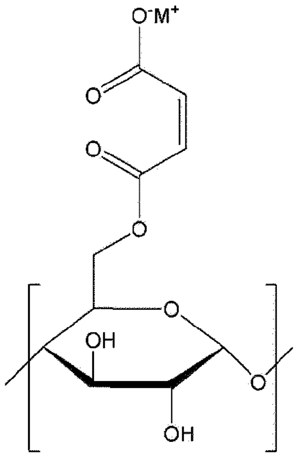

**1-1**

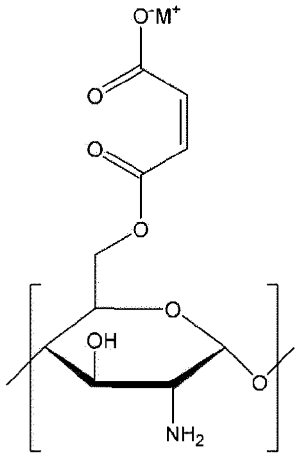

**1-2**

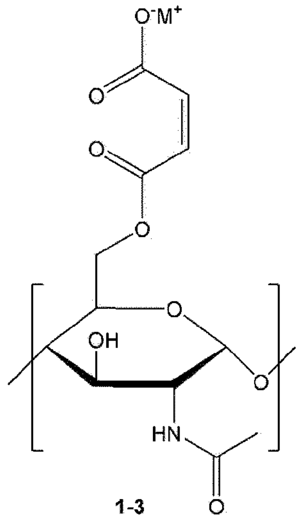

**1-3**

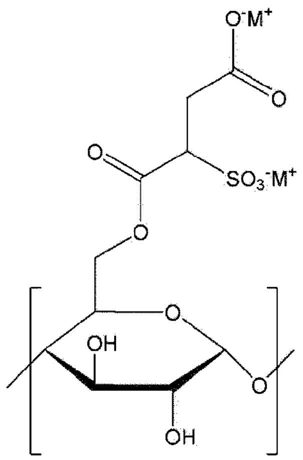

**2-1**

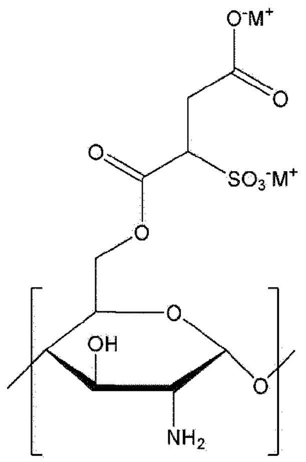

**2-2**

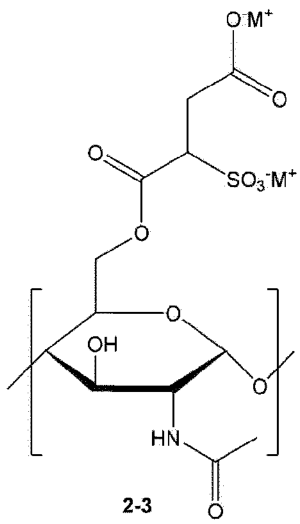

**2-3**

in Chemical Formulas 1-1 to 1-3 and 2-1 to 2-3, each M is independently hydrogen or an alkali metal.

Specifically, in Chemical Formulas 1-1 to 1-3 and 2-1 to 2-3, each $M^+$ is independently $H^+$, $Na^+$, or $K^+$.

In one example, when the modified polysaccharide is modified starch or modified dextrin, it may include the repeating units represented by Chemical Formula 1-1 and Chemical Formula 2-1, and when the modified polysaccharide is modified chitosan, it may include all of the repeating units represented by Chemical Formulas 1-2, 1-3, 2-1, and 2-2.

Further, the modified polysaccharide has a weight average molecular weight of 500 to 1,000,000 g/mol. When the weight average molecular weight of the modified starch is too low, there is a problem that sufficient crosslinking cannot be performed and a large amount of unreacted denatured starch remains, and when the weight average molecular weight is too high, an entanglement phenomenon of the polymer chains of modified polysaccharides with high molecular weight may occur, which makes acid treatment and functional group introduction itself difficult. Thus, there is a problem

that the production itself of a modified polysaccharide having a weight average molecular weight of more than 1,000,000 g/mol is not easy.

Here, the weight average molecular weight (Mw) can be measured by gel permeation chromatography (GPC) using polystyrene (PS) using a calibration standard sample. More specifically, 200 mg of the modified polysaccharide is diluted in 200 ml of dimethylformamide (DMF) solvent to prepare a sample of about 1000 ppm. Then, the weight average molecular weight can be measured with an RI detector at 1ml/min flow using Agilent 1200 series GPC instrument. At this time, the molecular weight of the sample can be calculated based on a calibration curve drawn up by using eight PS standards.

Meanwhile, the modified polysaccharide may be modified starch. The modified starch may include amylose and amylopectin in a weight ratio of 1:99 to 50:50 based on the total weight. Modification in which the hydroxyl group bonded to the 6th carbon of the glucose repeating unit is substituted with another substituent may occur in both amylose and amylopectin; However, from the viewpoint of processability and solubility, it is advantageous that the content of amylopectin is equal to or higher than the amylose content.

Further, the modified starch may have a gelatinization temperature of 50 to 90°C, and a peak viscosity (BU) of 50 to 1,000.

The modified starch may be a starch in which at least one starch composed of potato starch, corn starch, rice starch, wheat starch, tapioca starch and sweet potato starch has been modified. In particular, potato starch having a high content of amylopectin may be preferable from the viewpoint of processability and solubility.

Meanwhile, the monomer may further include an acrylic acid-based compound having an acidic group of which at least a part is neutralized.

The acrylic acid-based monomer is a compound represented by the following Chemical Formula 3:

[Chemical Formula 3] R-COOM'

wherein, in Chemical Formula 2,

R is an alkyl group containing an unsaturated bond and having 2 to 5 carbon atoms, and
M' is a hydrogen atom, a monovalent or divalent metal, an ammonium group, or an organic amine salt.

Preferably, the monomer may be one or more selected from the group consisting of (meth)acrylic acids, and a monovalent (alkali) metal salt, a divalent metal salt, an ammonium salt and an organic amine salt thereof.

When (meth)acrylic acid and/or a salt thereof is used as the acrylic acid-based monomer, a super absorbent polymer having improved absorbency can be obtained, which is thus advantageous.

Here, the acrylic acid-based monomer has acidic groups, wherein at least a part of the acidic group may be neutralized. Preferably, those partially neutralized with an alkali substance such as sodium hydroxide, potassium hydroxide, ammonium hydroxide or the like may be used. In this case, the acrylic acid-based monomer may have a degree of neutralization in the range of about 40 to 95 mol%, preferably about 40 to 80 mol%, and more preferably about 45 to 75 mol%. The range of the degree of neutralization may vary depending on the final physical properties. However, if the degree of neutralization is too high, the neutralized monomer is precipitated and thus the polymerization may not proceed smoothly. On the contrary, if the degree of neutralization is too low, not only the absorbency of the polymer is greatly reduced, but also the properties like elastic rubber that are difficult to handle can be exhibited.

Further, the term 'internal crosslinking agent' as used herein is a term used to distinguish it from a surface crosslinking agent commonly used for crosslinking the surface of super absorbent polymer particles, and serves to crosslink and polymerize the vinyl group of the above-mentioned modified polysaccharide. The crosslinking in the above step proceeds without surface or internal classification, but when the surface crosslinking process of the super absorbent polymer particles proceeds, the surface of the particles of the finally produced super absorbent polymer is composed of a structure crosslinked by a surface crosslinking agent, and the inside is composed of a structure crosslinked by the internal crosslinking agent.

As the internal crosslinking agent, any compound can be used as long as it enables the introduction of crosslinking during polymerization of the modified polysaccharide. Non-limiting examples of the internal crosslinking agent may include multifunctional cross-linking agents, such as N,N'methylenebisacrylamide, trimethylolpropane tri(meth)acrylate, ethylene glycol di(meth)acrylate, polyethylene glycol (meth)acrylate, polyethylene glycol di(meth)acrylate, propylene glycol di(meth)acrylate, polypropylene glycol(meth)acrylate, butanediol di(meth)acrylate, butylene glycol di(meth)acrylate, diethylene glycol di(meth)acrylate, hexanediol di(meth)acrylate, triethylene glycol di(meth)acrylate, tripropylene glycol di(meth)acrylate, tetraethylene glycol di(meth)acrylate, dipentaerythritol pentaacrylate, glycerin tri(meth)acrylate, pentaerythritol tetraacrylate, triarylamine, ethylene glycol diglycidyl ether, propylene glycol, glycerin, or ethylene carbonate, which can be used alone or in combination of two or more thereof, but are not limited thereto. Preferably, among them, N,N'-methylenebisacrylamide or polyethylene glycol diacrylate may be used

Crosslinking polymerization of the modified starch in the presence of such an internal crosslinking agent may be performed by thermal polymerization, photopolymerization or hybrid polymerization in the presence of a polymerization initiator, optionally a thickener, plasticizer, storage stabilizer, antioxidant, and the like, and detailed contents thereof will be described later.

Further, the super absorbent polymer may be in the form of particles having an average particle diameter of 150 to 850 μm. In this case, the particle size can be measured according to EDANA (European Disposables and Nonwovens Association) recommended test method No. WSP 220.3. More specifically, in the super absorbent polymer composition, about 90% by weight, preferably 95% by weight or more, based on the total weight, may be super absorbent polymer particles having a particle diameter of about 150 to 850 μm, and less than about 10% by weight, more specifically less than about 5% by weight, may be fine powders having a particle diameter of less than about 150 μm. When the super absorbent polymer contains a large amount of fine powders having a particle diameter of less than 150 μm, it may reduce various physical properties of the super absorbent polymer, which is thus not preferable.

Further, the biodegradable superb absorbent polymer may have a centrifuge retention capacity (CRC) of 10 to 50 g/g as measured according to EDANA recommended test method WSP 241.3.

Further, the biodegradable super absorbent polymer may have an absorbency under pressure (AUP) at 0.7 psi of 5 to 30 g/g as measured according to EDANA recommended test method WSP 242.3.

**Method for producing biodegradable super absorbent polymer**

Meanwhile, the biodegradable super absorbent polymer can be prepared by the following production method:
A method for producing a biodegradable super absorbent polymer, the method comprising the steps of:

preparing a monomer composition including a modified polysaccharide having a maleic acid group (-OCOCH=CHCOOH) and a sulfosuccinic acid group (-OCOCH($SO_3$H)$CH_2$COOH) in the presence of an internal crosslinking agent and a polymerization initiator;
subjecting the monomer composition to crosslinking polymerization to produce a hydrogel polymer;
drying, pulverizing and classifying the hydrogel polymer,
wherein at least a part of the maleic acid group and the sulfosuccinic acid group is neutralized,
the degree of substitution of maleic acid group in the modified polysaccharide is 0.15 to 0.65, and
the degree of substitution of sulfosuccinic acid group in the modified polysaccharide is 0.05 to 0.60.

Meanwhile, the modified polysaccharide can be produced by including:

a first step of acid-treating the unmodified polysaccharide;
a second step of reacting the acid-treated polysaccharide prepared in the first step with maleic acid or maleic acid anhydride; and
a third step of reacting the polysaccharide prepared in the second step with a sulfite-based compound.

First, in the first step for preparing the modified polysaccharide, the unmodified polysaccharide can be acid-treated. Such acid treatment destroys the rigid structure of starch by hydrogen bonding, and thus proceeds in order to increase the production efficiency for modification of polysaccharides such as maleation in the second step and sulfonation in the third step, which will be described later. Specifically, the acid treatment can be performed using an acidic solution such as hydrochloric acid at a temperature of 25 to 50°C for 6 to 48 hours.

Next, a second step of preparing a polysaccharide into which a maleic acid group has been introduced can be performed by reacting the polysaccharide acid-treated in the first step with maleic acid or maleic acid anhydride. Further, the reaction of the acid-treated polysaccharide in the second step with maleic acid or maleic acid anhydride can proceed at a temperature of 50 to 100°C for 4 to 12 hours.

In one example, the acid-treated polysaccharide can be maleated using maleic anhydride, whereby a maleic acid group-substituted polysaccharide can be prepared.

Next, the polysaccharide prepared in the second step can be reacted with a sulfite-based compound to prepare a polysaccharide into which a sulfonic acid group is further introduced in addition to a carboxyl group and a vinyl group. At this time, as the sulfite-based compound, one or more compounds selected from the group consisting of sodium bisulfite, potassium bisulfite, ammonium sulfite and sodium sulfite can be used. Further, specifically, the reaction of the polysaccharides prepared in the second step and the third step with the sulfite-based compound can be performed at a temperature of 30 to 60°C for 4 to 12 hours.

In addition, the sulfite-based compound can react with a vinyl group introduced into the polysaccharide.

In one example, the maleic acid group in the polysaccharide can be sulfonated using the sulfite-based compound, that is, the maleic acid group-substituted polysaccharide can be sulfosuccinylated, whereby a polysaccharide in which

a maleic acid group and a sulfosuccinic acid group are substituted can be prepared.

Next, a step of subjecting the prepared modified starch to crosslinking polymerization in the presence of an internal crosslinking agent and a polymerization initiator to form a hydrogel polymer can be performed.

The step may be composed of a step of preparing a monomer composition by mixing the modified starch, an internal crosslinking agent and a polymerization initiator, and a step of thermally polymerizing or photopolymerizing the monomer composition to form a hydrogel polymer. In this case, for detailed contents of the modified starch and the internal crosslinking agent, refer to the above.

Furthermore, the monomer composition may further include an acrylic acid-based compound having an acidic group of which at least a part is neutralized. In the monomer composition, the modified polysaccharide and the acrylic acid-based compound may be included in a weight ratio of 99:1 to 1:99. When the above-mentioned range is satisfied, the biodegradable super absorbent polymer can exhibit excellent biodegradability, and at the same time have improved absorbency and centrifuge retention capacity.

In the monomer composition, the internal crosslinking agent may be used in an amount of 0.1 to 5 parts by weight based on 100 parts by weight of the modified polysaccharide. For example, the internal crosslinking agent may be used in an amount of 0.1 parts by weight or more, or 0.2 parts by weight or more, and 5 parts by weight or less, 3 parts by weight or less, 2 parts by weight or less, 1 part by weight or less, or 0.5 parts by weight or less, based on 100 parts by weight of the modified polysaccharide. If the content of the internal crosslinking agent is too low, crosslinking does not occur sufficiently, and it may be difficult to achieve strength above an appropriate level. If the content of the internal crosslinking agent is too high, the internal crosslinking density may increase, which may make it difficult to achieve a desired centrifuge retention capacity.

Further, the polymerization initiator may be appropriately selected depending on the polymerization method. In the case of using a thermal polymerization method, a thermal polymerization initiator is used, in the case of using a photopolymerization method, a photopolymerization initiator is used, and in the case of using a hybrid polymerization method (a method using both heat and light), both a thermal polymerization initiator and a photopolymerization initiator can be used. However, even in the case of performing the photopolymerization method, a certain amount of heat is generated by light irradiation such as ultraviolet irradiation. Further, a certain amount of heat may be generated with the progress of the polymerization reaction, which is an exothermic reaction, a thermal polymerization initiator can be further used.

As the photopolymerization initiator, a compound capable of forming radicals by a light such as UV can be used without limitations in the constitution.

For example, one or more selected from the group consisting of benzoin ether, dialkyl acetophenone, hydroxyl alkylketone, phenyl glyoxylate, benzyl dimethyl ketal, acyl phosphine, and $\alpha$-aminoketone can be used as the photopolymerization initiator. Meanwhile, specific examples of acyl phosphine may include diphenyl(2,4,6-trimethylbenzoyl)phosphine oxide, phenylbis(2,4,6-trimethylbenzoyl)phosphine oxide, ethyl(2,4,6-trimethylbenzoyl)phenylphosphinate, and the like. More various photopolymerization initiators are well disclosed in "UV Coatings: Basics, Recent Developments and New Application (Elsevier, 2007)" written by Reinhold Schwalm, p 115, however, they are not limited to the above described examples.

Further, one or more selected from the group consisting of persulfate-based initiators, azo-based initiators, hydrogen peroxide and ascorbic acid can be used as the thermal polymerization initiator. Specific examples of the persulfate-based initiators may include sodium persulfate ($Na_2S_2O_8$), potassium persulfate ($K_2S_2O_8$), ammonium persulfate ($(NH4)_2S_2O_8$) or the like. Examples of the azo-based initiators may include 2,2-azobis(2-amidinopropane)dihydrochloride, 2,2-azobis-(N,N-dimethylene)isobutyramidine dihydrochloride, 2-(carbamoylazo)isobutylonitrile, 2,2-azobis(2-[2-imidazolin-2-yl]propane)dihydrochloride, 4,4-azobis-(4-cyanovaleric acid) or the like. More various thermal polymerization initiators are well-disclosed in Principle of Polymerization (Wiley, 1981)' written by Odian, p 203, however, they are not limited to the above described examples.

The polymerization initiator may be used at a concentration of 2 parts by weight or less based on 100 parts by weight of the modified polysaccharide. That is, when the concentration of the polymerization initiator is too low, the polymerization rate may become slow and thus a large amount of residual monomer may be extracted from the final product, which is thus not preferable. On the contrary, if the concentration of the polymerization initiator is too high, a polymer chain making up a network may become short, and thus, the physical properties of polymer may be degraded, such as increase in the content of water-soluble components and decrease in absorbency under pressure, which is not preferable.

The monomer composition may further include additives such as a thickener, a plasticizer, a storage stabilizer, and an antioxidant, if necessary.

Further, the monomer composition containing the monomer may be in a suspension state when it is insoluble like starch having a high molecular weight, and may be in a solution state dissolved in a solvent such as water when it is water-soluble like dextrin having a low molecular weight. The solid content in the monomer composition, that is, the concentration of the monomer, the internal crosslinking agent and the polymerization initiator can be appropriately

adjusted in consideration of the polymerization time, the reaction conditions and the like. For example, the solids content in the monomer composition may be 10 to 80% by weight, or 15 to 60% by weight, or 30 to 50% by weight.

When the monomer composition has a solid content in the above range, it is not necessary to remove unreacted monomers after polymerization by using the gel effect phenomenon that appears in the polymerization reaction of a high-concentration aqueous solution, and at the same time, it may be advantageous to adjust the pulverization efficiency at the time of pulverization of the polymer described later.

In this case, as the usable solvent, any solvent may be used without limitations in the constitution, as long as it is able to dissolve the above-mentioned components. For example, the solvent may include one or more selected from water, ethanol, ethylene glycol, diethylene glycol, triethylene glycol, 1,4-butanediol, propylene glycol, ethylene glycol monobutyl ether, propylene glycol monomethyl ether, propylene glycol monomethyl ether acetate, methyl ethyl ketone, acetone, methyl amyl ketone, cyclohexanone, cyclopentanone, diethylene glycol monomethyl ether, diethylene glycol ethylether, toluene, xylene, butyrolactone, carbitol, methyl cellosolve acetate, N,N-dimethylacetamide, or a mixture thereof.

Meanwhile, the crosslinking polymerization of the modified polysaccharide can proceed without particular limitation, as long as the hydrogel polymer can be formed by thermal polymerization, photopolymerization, or hybrid polymerization.

Specifically, the polymerization method is largely classified into the thermal polymerization and the photopolymerization according to the polymerization energy source. Usually, the thermal polymerization may be carried out in a reactor like a kneader equipped with agitating spindles and the photo-polymerization may be carried out in a reactor equipped with a movable conveyor belt. The above-described polymerization method is an example only, and the present disclosure is not limited to the above-described method.

For example, as described above, thermal polymerization is performed by providing hot air to a reactor like a kneader equipped with the agitating spindles or by heating the reactor so as to obtain the hydrogel polymer. The obtained hydrogel polymer may have the size of centimeters or millimeters when it is discharged from the outlet of the reactor, according to the type of agitating spindles equipped in the reactor. Specifically, the size of the obtained hydrogel polymer may appear in various forms according to the concentration of the monomer composition fed thereto, the feeding speed or the like, and the hydrogel polymer having a weight average particle size of 2 to 50 mm may be generally obtained.

Further, as described above, when the photopolymerization is carried out in a reactor equipped with a movable conveyor belt, the hydrogel polymer typically obtained may be a hydrogel polymer in a sheet-type having a width of the belt. In this case, the thickness of the polymer sheet may vary according to the concentration of the monomer composition fed thereto and the feeding speed or the feeding amount. However, the monomer composition is preferably fed so as to obtain a sheet-type polymer having a thickness of about 0.5 to about 5 cm. If the monomer composition is fed so that the thickness of the sheet-type polymer becomes too thin, the production efficiency becomes low, which is not preferred. If the thickness of the sheet-type polymer exceeds 5 cm, the polymerization reaction may not uniformly occur throughout the polymer due to the excessively high thickness.

In this case, the hydrogel polymer obtained by the above method may have typically a water content of about 40 to about 70% by weight. For example, the water content of the hydrogel polymer may be 40% by weight or more, 45% by weight or more, or 50% by weight or more, and 70% by weight or less, 65% by weight or less, or 60% by weight or less. When the water content of the hydrogel polymer is too low, it becomes difficult to secure an appropriate surface area in the subsequent pulverization step, and the drying efficiency may decrease. When the water content of the hydrogel polymer is too high, the pressure received in the subsequent pulverization step may increase, so that the absorbency under pressure may be lowered, which may require a lot of energy and a long time in the drying step after pulverization.

Meanwhile, the term "water content" as used herein means a weight occupied by water with respect to a total weight of the hydrogel polymer, which may be the value obtained by subtracting the weight of the dried polymer from the weight of the hydrogel polymer. Specifically, the water content is defined as a value calculated by measuring the weight loss due to evaporation of water in the polymer in the process of drying by raising the temperature of the polymer in a crumb state through infrared heating. At this time, the drying for measuring the water content is performed by raising the temperature from room temperature to about 50°C and then vacuum drying at 50°C for about 6 hours.

Meanwhile, after the production of the hydrogel polymer, a coarse pulverization step of pulverizing the produced hydrogel polymer before the subsequent drying and pulverization steps can be selectively performed.

The coarse pulverization step is a step for increasing drying efficiency in a subsequent drying step and controlling the particle size of the finally produced super absorbent polymer powder. At this time, a pulverizing machine used herein may include, but its configuration is not limited to, for example, any one selected from the group consisting of a vertical pulverizer, a turbo cutter, a turbo grinder, a rotary cutter mill, a cutter mill, a disc mill, a shred crusher, a crusher, a chopper, and a disc cutter. However, the present disclosure is not limited to the above-described examples.

The coarse pulverizing step can be performed so that the hydrogel polymer has a particle size of about 2 to about 10 mm. Pulverizing the hydrogel polymer into a particle size of less than 2 mm is technically not easy due to a

high water content of the hydrogel polymer, and a phenomenon of agglomeration may occur between the pulverized particles. Meanwhile, if the hydrogel polymer is pulverized into a particle size of larger than 10 mm, the effect of increasing the efficiency in the subsequent drying step may be insignificant.

Next, the steps of drying, pulverizing, and classifying the hydrogel polymer produced in the above step to produce a base polymer is performed.

The drying method may be selected and used without limitation in the constitution if it is a method commonly used in the relevant art. Specifically, the drying step may be carried out by a method such as hot air supply, infrared irradiation, microwave irradiation or ultraviolet irradiation.

Specifically, the drying can be performed under vacuum conditions at a temperature of less than 100°C, specifically, a temperature of about 30°C to about 80°C. When the drying temperature is 100°C or higher, the modified polysaccharide can be decomposed, which is thus not suitable. When the drying temperature is less than 30°C, the drying time may be too long.

Meanwhile, the drying time may proceed for about 20 to about 90 minutes, in consideration of the process efficiency and the like, but it is not limited thereto.

After the drying step, the water content of the polymer may be about 5 to about 10% by weight.

After the drying step, a pulverization step is performed.

The pulverization step can be performed so that the particle diameter of the polymer powder, that is, the base polymer, is about 150 to about 850 μm. Specific examples of a pulverizing device that can be used to pulverize into the above particle diameter may include a pin mill, a hammer mill, a screw mill, a roll mill, a disc mill, a jog mill or the like, but it is not limited to the above-described examples.

Further, in order to control the physical properties of the super absorbent polymer powder finally produced after the pulverization step as described above, a step of classifying the pulverized polymer powder depending on the particle diameter may be further undergone.

Preferably, a polymer having a particle diameter of about 150 to about 850 μm is classified and only the polymer powder having such a particle diameter can be used as a base polymer to undergo a surface crosslinking reaction, which can be finally commercialized.

The base polymer obtained as a result of the above process may have a powder form including an acrylic acid-based monomer and a crosslinked polymer crosslinked through an internal crosslinking agent. Specifically, the base polymer may have a powder form having a particle diameter of 150 to 850 μm.

On the other hand, there is further provided a hygiene product comprising the above-mentioned biodegradable super absorbent polymer.

Hereinafter, preferred embodiments are presented to facilitate the understanding of the invention. However, the following examples are for illustrative purposes only and are not intended to limit the present disclosure.

Production Example 1: Production of modified chitosan (M/S)

(1) Step 1: Maleation step of chitosan
20 g of chitosan and 100 g of maleic anhydride were put into a 250 ml three-necked flask, and then stirred at 90°C for 6 hours. After the reaction, the mixture was precipitated in acetone solvent and filtered to remove excess maleic anhydride, which was dried at 50°C for 24 hours or more to obtain maleated chitosan.

(2) Step 2: Sulfonation of at least part of maleated functional groups
20 g of maleated chitosan and 5 g of sodium bisulfite were put into a 250 ml three-necked flask, and then stirred at 40°C for 6 hours. After stirring, the reaction mixture was adjusted to pH 6-7 by adding $Na_2CO_3$, washed several times with water and ethanol, and then dried at 50°C for 24 hours or more to finally produce modified chitosan (M/S) into which maleic acid group (-OCOCH=CHCOOH) and sulfosuccinic acid group ($-OCOCH(SO_3H)CH_2COOH$) were introduced.

The weight average molecular weight of the produced chitosan was 50,000 g/mol, the degree of substitution ($DS_M$) of maleic acid group (-OCOCH=CHCOOH) was 0.22, and the degree of substitution (DSs) of sulfosuccinic acid group ($-OCOCH(SO_3H)CH_2COOH$) was 0.55. At this time, the degree of substitution of each of these substituent groups was calculated based on the integral ratio of the peak corresponding to vinyl (CH=CH, 6.3 ppm) of the maleic acid group in $^1H$ NMR, and the specific calculation method is as follows.

First, a $^1H$ NMR spectrum of maleated chitosan produced in step 1 was obtained, which is shown in Fig. 1. Referring to Fig. 1, it can be confirmed that a maleic acid group has been introduced into chitosan, in light of the fact that a peak appeared at 6.3 ppm corresponding to the vinyl group of the maleic acid group. Based on the integral ratio

of these peaks, it was possible to confirm the degree of substitution of the maleic acid group (-OCOCH=CHCOOH) of the maleated chitosan produced in step 1.

Next, $^1$H NMR spectrum was obtained for the finally produced modified chitosan (M/S), which is shown in Fig. 2. Referring to Fig. 2, in light of the decrease in the peak at 6.3 ppm corresponding to the vinyl group of the maleic acid group, it was confirmed that the sulfonic acid group was introduced into the vinyl group of the maleic acid group and thus, the vinyl group of the maleic acid group was reduced. Further, based on the integral ratio of these peaks, it was possible to confirm the degree of substitution of the maleic acid group (-OCOCH=CHCOOH) of the finally produced modified chitosan (M/S). Further, as a result, it was possible to calculate the degree of substitution of the sulfosuccinic acid group (-OCOCH($SO_3$H)$CH_2$COOH) of the finally produced modified chitosan (M/S).

**Production Example 2: Production of modified starch 1 (M/S-1)**

(1) Step 1: Acid treatment of unmodified starch
20 g of starch was added to 200 ml of 2M HCl solution and then stirred at 35°C for 24 hours. Then, the mixture was filtered, washed several times with water and ethanol, and then dried at 50°C for 24 hours or more to obtain acid-treated starch.

(2) Step 2: **Maleation of acid-treated starch**
20 g of acid-treated starch and 100 g of maleic anhydride were put into a 250 ml three-necked flask, and then stirred at 90°C for 6 hours. After the reaction, the mixture was precipitated in acetone solvent and filtered to remove excess maleic anhydride, which was dried at 50°C for more than 24 hours. Thereby, maleated modified starch was obtained.

(3) Step 3: Sulfonation of at least part of maleated functional groups
20 g of maleated modified starch and 5 g of sodium bisulfite were put into a 250 ml three-necked flask, and then stirred at 40°C for 6 hours. After stirring, the reaction mixture was adjusted to pH 6~7 by adding $Na_2CO_3$, washed several times with water and ethanol, and then dried at 50°C for 24 hours or more to finally produce modified starch 1 (M/S-1) into which maleic acid group (-OCOCH=CHCOOH) and sulfosuccinic acid group (-OCOCH($SO_3$H)$CH_2$COOH) was introduced.

The produced modified starch has a weight average molecular weight of 50,000 g/mol, the degree of substitution ($DS_M$) of maleic acid group (-OCOCH=CHCOOH) of 0.62, and the degree of substitution (DSs) of sulfosuccinic acid group (-OCOCH($SO_3$H)$CH_2$COOH) of 0.07.

At this time, $^1$H NMR spectra of each of the maleated modified starch produced in step 2 and the finally produced modified starch (M/S) were obtained, and then the degree of substitution of each substituent group was calculated based on the integral ratio of the peak corresponding to the vinyl (CH=CH, 6.3ppm) of the maleic acid group in each spectrum. For a detailed calculation method, refer to the calculation method of the degree of substitution for the modified chitosan (M / S) of Production Example 1. Hereinafter, the rest of the Production Examples were calculated in the same manner.

**Production Example 3: Production of modified starch** 2 (M/S-2)

Modified starch 2 (M/S-2) was produced using the same method as in Production Example 2. except that 10 g of sodium bisulfite was used in Production Example 2.

The produced modified starch had a weight average molecular weight of 50,000 g/mol, a degree of substitution ($DS_M$) of maleic acid group (-OCOCH=CHCOOH) of 0.51, and a degree of substitution (DSs) of sulfosuccinic acid group (-OCOCH($SO_3$H)$CH_2$COOH) of 0.16.

**Production Example 4: Production of modified starch 3 (M/S-3)**

Modified starch 3 (M/S-3) was produced using the same method as in Production Example 2, except that 14 g of sodium bisulfite was used in Production Example 2.

The produced modified starch had a weight average molecular weight of 50,000 g/mol, a degree of substitution ($DS_M$) of maleic acid group (-OCOCH=CHCOOH) of 0.36, and a degree of substitution (DSs) of sulfosuccinic acid group (-OCOCH($SO_3$H)$CH_2$COOH) of 0.28.

**Production Example 5: Production of modified starch 4 (M/S-4)**

Modified starch 4 (M/S-4) was produced using the same method as in Production Example 2, except that 20 g of sodium bisulfite was used in Production Example 2.

The produced modified starch had a weight average molecular weight of 50,000 g/mol, a degree of substitution ($DS_M$) of maleic acid group (-OCOCH=CHCOOH) of 0.21, and a degree of substitution (DSs) of sulfosuccinic acid group ($-OCOCH(SO_3H)CH_2COOH$) of 0.43.

**Comparative Production Example** 1: **Production of modified starch (M)**

(1) Step 1: Acid treatment of unmodified starch
20 g of starch was added to 200 ml of 2M HCl solution and then stirred at 35°C for 24 hours. Then, the mixture was filtered, washed several times with water and ethanol, and then dried at 50°C for 24 hours or more to obtain acid-treated starch.

(2) Step 2: Maleation of acid-treated starch
20 g of acid-treated starch and 100 g of maleic anhydride were put into a 250 ml three-necked flask, and then stirred at 90°C for 6 hours. After the reaction, the mixture was precipitated in an acetone solvent and filtered to remove excess maleic anhydride, which was dried at 50°C for 24 hours or more. Thereby, maleated modified starch (M) was obtained.

The finally produced modified starch (M) had a weight average molecular weight of 50,000 g/mol and a degree of substitution ($DS_M$) of maleic acid group (-OCOCH=CHCOOH) of 0.70.

Comparative Production Example 2: Production of modified starch **(M/S')**

Modified starch (M/S') was produced using the same method as in Production Example 2, except that 30 g of sodium bisulfite was used in Production Example 2.

The produced modified starch had a weight average molecular weight of 50,000 g/mol, a degree of substitution ($DS_M$) of maleic acid group (-OCOCH=CHCOOH) of 0.14, and a degree of substitution (DSs) of sulfosuccinic acid group ($-OCOCH(SO_3H)CH_2COOH$) of 0.54.

Example 1

In a 3L glass vessel equipped with a stirrer, nitrogen injector and thermometer, 100 g of modified chitosan (M/S) produced in Production Example 1, 0.23 g of polyethylene glycol diacrylate (Mn = 575) as an internal crosslinking agent, 0.008 g of bis(2,4,6-trimethylbenzoyl)-phenylphosphine oxide as a photoinitiator, 0.12 g of sodium persulfate (SPS) as a thermal initiator, 39.7 g of 98% sodium hydroxide solution and 200 g of water were added to prepare a monomer composition while continuously feeding nitrogen.

The monomer composition was added to a stainless steel container having a width of 250 mm, a length of 250 mm, and a height of 30 mm, and UV light was irradiated for 60 seconds in a UV chamber at 80°C (irradiation dose: 10 $mV/cm^2$) and aged for 2 minutes to obtain a hydrogel polymer. The obtained hydrogel polymer was pulverized to a size of 3 mm * 3 mm, and then the obtained gel-type polymer was spread on a stainless wire gauze having a pore size of 600 $\mu$m to a thickness of about 30 mm, and dried in a vacuum oven at 50°C for 10 hours. The dried polymer thus obtained is pulverized using a pulverizing device, and classified through a standard mesh sieve of ASTM standard to obtain a base polymer having a particle size of 300 to 600 $\mu$m, which was used as a super absorbent polymer.

**Examples 2 to 5**

A super absorbent polymer was produced using the same method as in Example 1, except that the modified starch of Table 1 below was used instead of the modified chitosan (M/S).

**Comparative Examples 1 and 2**

A super absorbent polymer was produced using the same method as in Example 1, except that the modified starch of Table 1 below was used instead of the modified chitosan (M/S).

## Experimental Example

The physical properties of the super absorbent polymers produced in Examples and Comparative Examples were evaluated by the following method, and the results are shown in Table 1 below.

Unless otherwise specified, all of the following physical property evaluations were performed at constant temperature and humidity (23±1°C, relative humidity 50±10%). The physiological saline or saline means 0.9 wt.% sodium chloride (NaCl) aqueous solution.

(1) Centrifuge Retention Capacity (CRC)

The centrifuge retention capacity of each polymer by water absorption capacity under a non-loading condition was measured in accordance with EDANA (European Disposables and Nonwovens Association) recommended test method No. WSP 241.3.

Specifically, $W_0$ (g) (about 0.2 g) of the super absorbent polymer was uniformly put in a nonwoven fabric-made bag and sealed. Then, the bag was immersed in a physiological saline solution (0.9 wt%) at room temperature. After 30 minutes, water was removed from the bag by centrifugation at 250 G for 3 minutes, and the weight $W_2$(g) of the bag was then measured. In addition, the same procedure was carried out without using the polymer, and then the resultant weight $W_1$(g) was measured. Using the respective weights thus obtained, CRC (g/g) was calculated according to the following Equation:

[Mathematical Equation 1]

$$CRC\ (g/g) = \{[W_2(g) - W_1(g)]/W_0(g)\} - 1$$

**(2) Absorbency Under Pressure (AUP)**

The absorbency under pressure at 0.7 psi was measured for each polymer in accordance with EDANA recommended test method No. WSP 242.3.

Specifically, a 400 mesh stainless steel screen was installed in the bottom of the plastic cylinder having an internal diameter of 60 mm. The super absorbent polymer $W_0$(g)(0.90 g) was uniformly scattered on the steel screen at room temperature and humidity of 50%, and a piston capable of uniformly further providing a load of 0.7 psi thereon was slightly smaller than the outer diameter of 60 mm, had no gap with the internal wall of the cylinder, and set so that it was not interrupted to move up and down. At this time, the weight $W_3$(g) of the device was measured.

A glass filter having a diameter of 90 mm and a thickness of 5 mm was placed in a Petri dish having a diameter of 150 mm, and then a physiological saline solution composed of 0.9% by weight of sodium chloride was poured in the Petri dish until the surface level became equal to the upper surface of the glass filter. A sheet of filter paper having a diameter of 90 mm was put thereon. The measuring device was put on the filter paper and the solution was absorbed for 1 hour under the load. After 1 hour, the weight $W_4$(g) was measured after lifting up the measuring device.

Using the respective weights thus obtained, the absorbency under pressure (g/g) was calculated according to the following Equation.

[Mathematical Equation 2]

$$AUP(g/g) = [W_4(g) - W_3(g)]/W_0(g)$$

(3) Biodegradability test

It was measured according to KS M ISO 14855-1 standard.

[Table 1]

| | Modified polysaccharide | $DS_M$ 1) | $DS_S$ 2) | CRC (g/g) | AUP (g/g) | Biodegra dability |
|---|---|---|---|---|---|---|
| Ex. 1 | Modified chitosan (M/S) | 0.22 | 0.55 | 4.2 | 3.3 | 96 % |
| Ex. 2 | Modified starch 1 (M/S-1) | 0.62 | 0.07 | 12.7 | 7.2 | 98 % |
| Ex. 3 | Modified starch 2(M/S-2) | 0.51 | 0.16 | 14.5 | 9.7 | 98 % |

(continued)

| | Modified polysaccharide | $DS_M$ 1) | $DS_S$ 2) | CRC (g/g) | AUP (g/g) | Biodegra dability |
|---|---|---|---|---|---|---|
| Ex. 4 | Modified starch 3(M/S-3) | 0.36 | 0.28 | 14.1 | 9.1 | 95 % |
| Ex. 5 | Modified starch 4(M/S-4) | 0.21 | 0.43 | 13.9 | 8.5 | 94 % |
| Comparat ive Ex. 1 | Modified starch (M) | 0.70 | 0 | 11.8 | 6.5 | 90 % |
| Comparat ive Ex. 2 | Modified starch (M/S') | 0.14 | 0.54 | 10.9 | 6.1 | 80 % |

1) $DS_M$: Degree of substitution of maleic acid groups in modified polysaccharide
2) DSs: Degree of substitution of sulfosuccinic acid group in modified polysaccharide

## Claims

**1.** A biodegradable super absorbent polymer comprising:

a crosslinked polymer of a monomer including a modified polysaccharide having a maleic acid group (-OCOCH=CHCOOH) and a sulfosuccinic acid group ($-OCOCH(SO_3H)CH_2COOH$), and an internal crosslinking agent,
wherein at least a part of the maleic acid group and the sulfosuccinic acid group is neutralized,
the degree of substitution of maleic acid group in the modified polysaccharide is 0.15 to 0.65, and
the degree of substitution of sulfosuccinic acid group in the modified polysaccharide is 0.05 to 0.60.

**2.** The biodegradable super absorbent polymer according to claim 1, wherein the modified polysaccharide is modified starch or modified chitosan.

**3.** The biodegradable super absorbent polymer according to claim 1, wherein the maleic acid group is introduced by maleic acid or maleic acid anhydride.

**4.** The biodegradable super absorbent polymer according to claim 1, wherein the sum of the degree of substitution of the maleic acid group and the degree of substitution of the sulfosuccinic acid group is 1 or less.

**5.** The biodegradable super absorbent polymer according to claim 1, wherein the ratio of the degree of substitution of the maleic acid group to the degree of substitution of the sulfosuccinic acid group is 1: 0.1 to 1: 3.0.

**6.** The biodegradable super absorbent polymer according to claim 1, wherein the modified polysaccharide comprises at least one of the repeating units represented by the following Chemical Formulas 1-1 to 1-3; and at least one of the repeating units represented by the following Chemical Formulas 2-1 to 2-3:

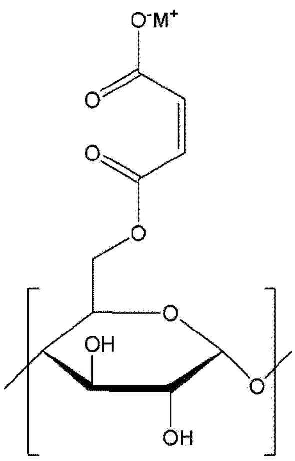
1-1

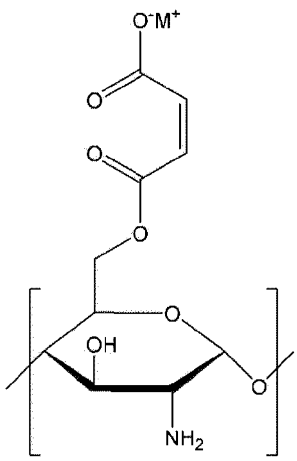
1-2

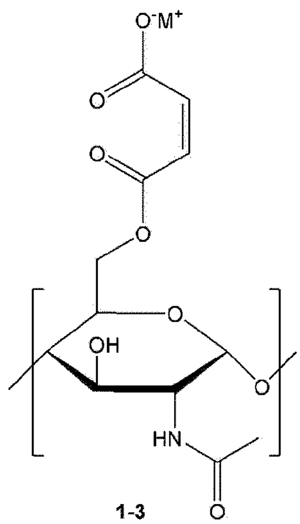
1-3

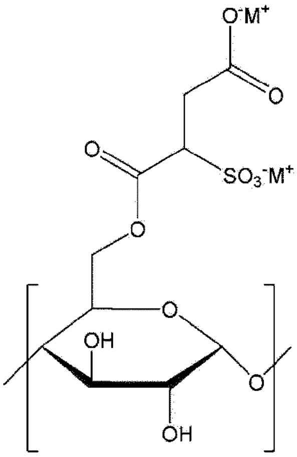

2-1

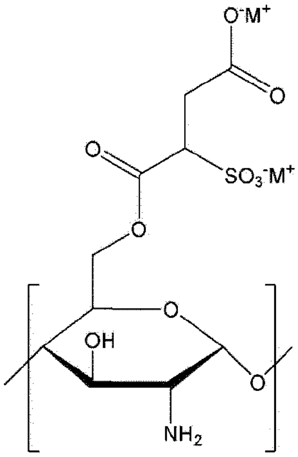

2-2

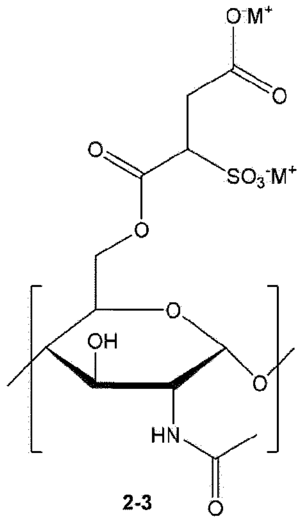

2-3

in Chemical Formulas 1-1 to 1-3 and 2-1 to 2-3,
each M is independently hydrogen or an alkali metal.

**7.** The biodegradable super absorbent polymer according to claim 1, wherein the monomer further comprises an acrylic acid-based compound having an acidic group of which at least a part is neutralized.

**8.** A method for producing a biodegradable super absorbent polymer, the method comprising the steps of:

preparing a monomer composition including a modified polysaccharide having a maleic acid group (-OCOCH=CHCOOH) and a sulfosuccinic acid group (-OCOCH($SO_3$H)$CH_2$COOH) in the presence of an internal crosslinking agent and a polymerization initiator;
subjecting the monomer composition to crosslinking polymerization to produce a hydrogel polymer;
drying, pulverizing and classifying the hydrogel polymer,
wherein at least a part of the maleic acid group and the sulfosuccinic acid group is neutralized,
the degree of substitution of maleic acid group in the modified polysaccharide is 0.15 to 0.65, and
the degree of substitution of sulfosuccinic acid group in the modified polysaccharide is 0.05 to 0.60.

**9.** The method according to claim 8, wherein the modified polysaccharide is produced by comprising:

a first step of acid-treating the unmodified polysaccharide;
a second step of reacting the acid-treated polysaccharide prepared in the first step with maleic acid or maleic acid anhydride; and
a third step of reacting the polysaccharide prepared in the second step with a sulfite-based compound.

**10.** The method according to claim 9, wherein in the third step, the sulfite-based compound reacts with a vinyl group introduced into the polysaccharide.

**11.** The method according to claim 8, wherein the monomer composition further comprises an acrylic acid-based compound having an acidic group of which at least a part is neutralized.

**12.** A hygiene product comprising the biodegradable super absorbent polymer according to any one of claims 1 to 7.

[FIG. 1]

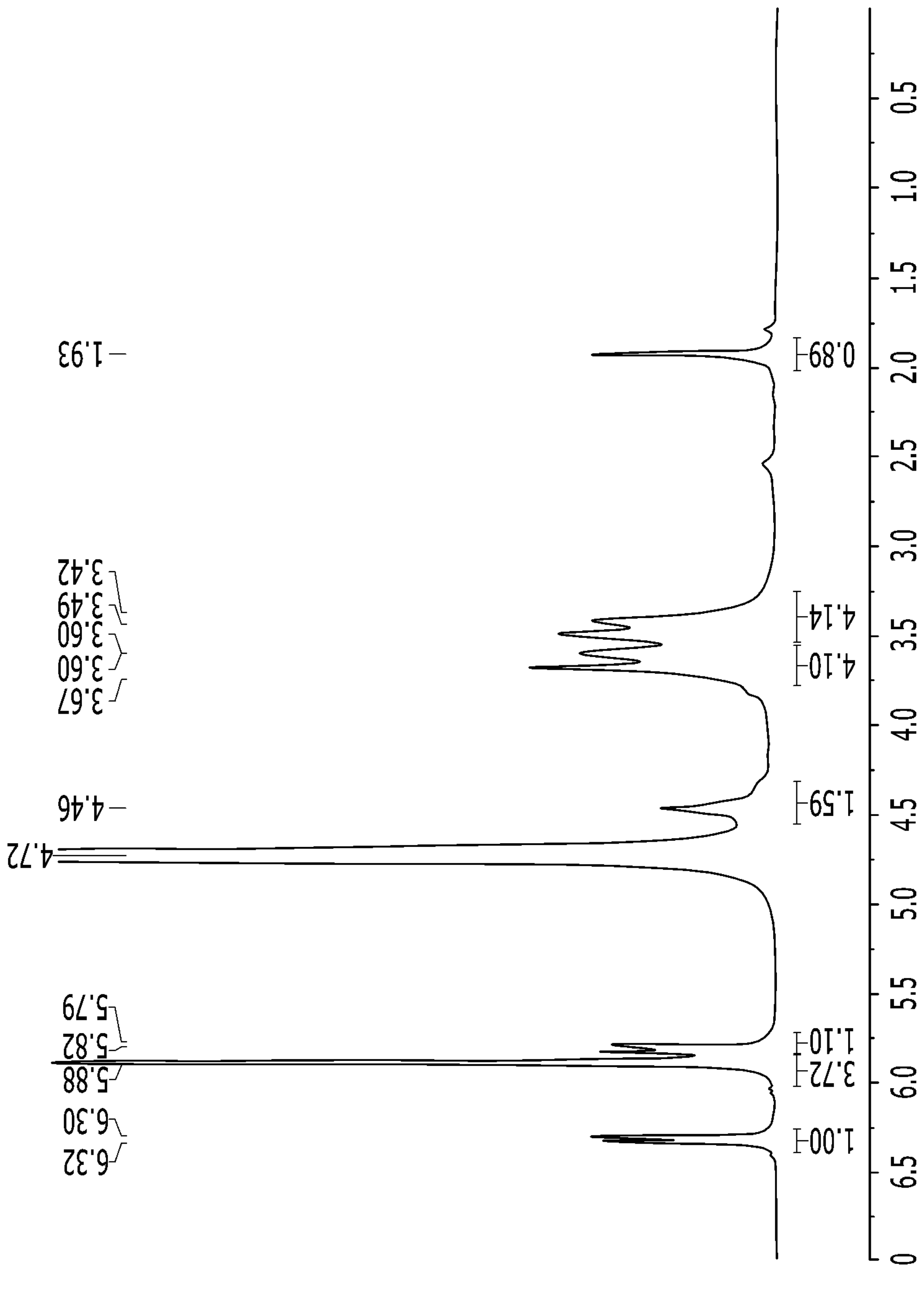
1.93
3.42
3.49
3.60
3.60
3.67
4.46
4.72
5.79
5.82
5.88
6.30
6.32
0.89
4.14
4.10
1.59
1.10
3.72
1.00
0.5
1.0
1.5
2.0
2.5
3.0
3.5
4.0
4.5
5.0
5.5
6.0
6.5
0

[FIG. 2]

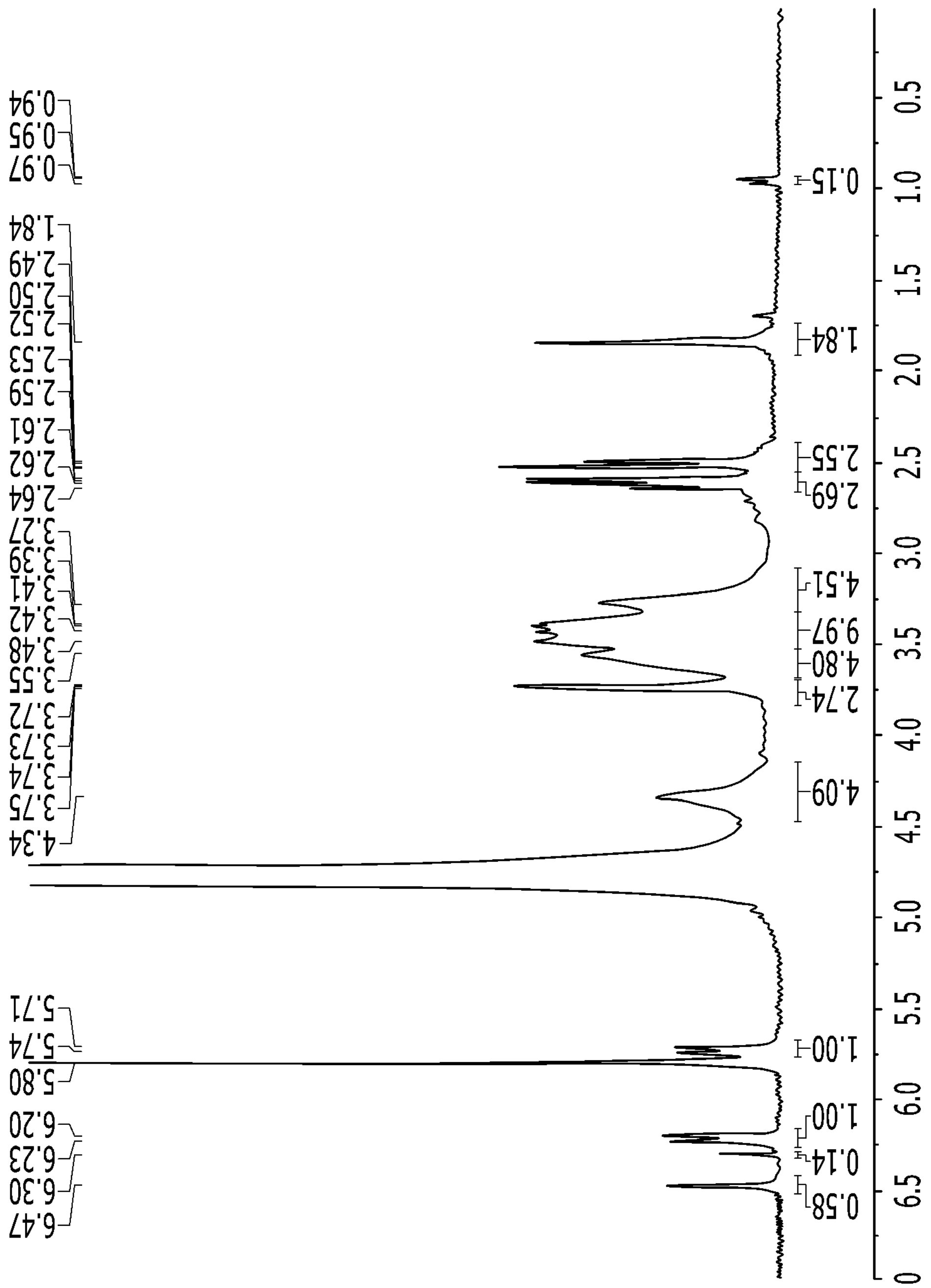
0.94
0.95
0.97
1.84
2.49
2.50
2.52
2.53
2.59
2.61
2.62
2.64
3.27
3.39
3.41
3.42
3.48
3.55
3.72
3.73
3.74
3.75
4.34
5.71
5.74
5.80
6.20
6.23
6.30
6.47
0.15
1.84
2.55
2.69
4.51
9.97
4.80
2.74
4.09
1.00
1.00
0.14
0.58
0.5
1.0
1.5
2.0
2.5
3.0
3.5
4.0
4.5
5.0
5.5
6.0
6.5

## INTERNATIONAL SEARCH REPORT

International application No. **PCT/KR2021/012862**

**A. CLASSIFICATION OF SUBJECT MATTER**

**C08F 251/00**(2006.01)i; **C08B 31/00**(2006.01)i; **C08B 37/08**(2006.01)i; **A61L 15/60**(2006.01)i

According to International Patent Classification (IPC) or to both national classification and IPC

**B. FIELDS SEARCHED**

Minimum documentation searched (classification system followed by classification symbols)

C08F 251/00(2006.01); C08F 220/06(2006.01); C08F 290/10(2006.01); C08G 73/10(2006.01); C08L 51/08(2006.01); C08L 67/02(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: 말레산기(maleic group), 술포숙신산기(sulfosuccinate grop), 폴리사카라이드(polysaccharide), 생분해성(degradable), 고흡수성 수지(super absorbent resin)

**C. DOCUMENTS CONSIDERED TO BE RELEVANT**

| Category* | Citation of document, with indication, where appropriate, of the relevant passages | Relevant to claim No. |
|---|---|---|
| A | CN 104558442 A (QINGDAO WENCHUANG TECHNOLOGY CO., LTD.) 29 April 2015 (2015-04-29)<br>See abstract; and claim 1. | 1-12 |
| A | HUANG, Y. et al. Preparation and characteristics of the sulfonated chitosan derivatives electrodeposited onto 316l stainless steel surface. JOURNAL OF BIOMATERIALS SCIENCE. POLYMER EDITION. 2017, vol. 29, no. 3, pp. 236-256 (inner pp. 1-20).<br>See abstract; and formula 1. | 1-12 |
| A | KR 10-2001-0082336 A (MITSUI CHEMICALS, INC.) 29 August 2001 (2001-08-29)<br>See entire document. | 1-12 |
| A | CN 107325502 A (SHAANXI UNIVERSITY OF SCIENCE AND TECHNOLOGY) 07 November 2017 (2017-11-07)<br>See entire document. | 1-12 |
| A | JEGAL, J. E. et al. Chitosan Membranes Crosslinked with Sulfosuccinic Acid for the Pervaporation Separation of Water/Alcohol Mixtures. Journal of Applied Polymer Science. 1999, vol. 71, pp. 671-675.<br>See entire document. | 1-12 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **10 January 2022** | **10 January 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**
**Information on patent family members**

International application No.
**PCT/KR2021/012862**

| Patent document cited in search report | Publication date (day/month/year) | Patent family member(s) | Publication date (day/month/year) |
|---|---|---|---|
| CN 104558442 A | 29 April 2015 | None | |
| KR 10-2001-0082336 A | 29 August 2001 | CN 1332765 A | 23 January 2002 |
| | | EP 1142925 A1 | 10 October 2001 |
| | | JP 2000-239378 A | 05 September 2000 |
| | | WO 00-39194 A1 | 06 July 2000 |
| CN 107325502 A | 07 November 2017 | CN 107325502 B | 26 March 2019 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020200125238

### Non-patent literature cited in the description

- **REINHOLD SCHWALM.** UV Coatings: Basics, Recent Developments and New Application. Elsevier, 2007, 115
- **ODIAN.** Principle of Polymerization. Wiley, 1981, 203